(19) Europäisches Patentamt
European Patent Office
Office européen des brevets

(11) **EP 2 223 683 B1**

(12) **EUROPEAN PATENT SPECIFICATION**

(45) Date of publication and mention
of the grant of the patent:
**18.06.2014 Bulletin 2014/25**

(21) Application number: **08866771.2**

(22) Date of filing: **26.12.2008**

(51) Int Cl.:
*A61K 9/14* (2006.01)     *A61K 9/16* (2006.01)
*A61K 31/44* (2006.01)    *A61K 31/513* (2006.01)
*A61K 31/53* (2006.01)    *A61K 47/10* (2006.01)
*A61K 47/26* (2006.01)    *A61P 35/00* (2006.01)

(86) International application number:
**PCT/JP2008/003991**

(87) International publication number:
**WO 2009/084216 (09.07.2009 Gazette 2009/28)**

(54) **ORAL PARTICULATE ANTITUMOR PREPARATION**

ORALE PARTIKELHALTIGE ANTITUMOR ZUSAMMENSETZUNG

PREPARATION ORALE PARTICULAIRE D'AGENT ANTITUMORAL

(84) Designated Contracting States:
**AT BE BG CH CY CZ DE DK EE ES FI FR GB GR
HR HU IE IS IT LI LT LU LV MC MT NL NO PL PT
RO SE SI SK TR**

(30) Priority: **27.12.2007 JP 2007335754**

(43) Date of publication of application:
**01.09.2010 Bulletin 2010/35**

(73) Proprietor: **Taiho Pharmaceutical Co., Ltd.
Chiyoda-ku, Tokyo 101-8444 (JP)**

(72) Inventors:
• **OHNISHI, Yoshito
Tokushima-shi
Tokushima 771-0132 (JP)**
• **OGATA, Tetsuo
Tokushima-shi
Tokushima 771-0132 (JP)**

(74) Representative: **Hartz, Nikolai
Wächtershäuser & Hartz
Patentanwaltspartnerschaft mbB
Weinstrasse 8
80333 München (DE)**

(56) References cited:
**EP-A1- 1 757 283        WO-A1-2004/087126
WO-A1-2005/120480    JP-A- 5 194 200
JP-A- 2003 034 632    JP-A- 2005 289 911
JP-A- 2007 045 781    JP-T- 2002 526 400
JP-T- 2005 506 323    JP-T- 2005 508 301
JP-T- 2006 516 597    JP-T- 2006 521 300**

• **ISHIKAWA T ET AL: "Improved survival with oral
administration of enteric-coated tegafur/uracil
for advanced stage IV-A hepatocellular
carcinoma.", JOURNAL OF
GASTROENTEROLOGY AND HEPATOLOGY
APR 2001 LNKD- PUBMED:11354285, vol. 16, no.
4, April 2001 (2001-04), pages 452-459,
XP002613411, ISSN: 0815-9319**
• **CARTEI G ET AL: "Oral 5-fluorouracil in
squamous cell carcinoma of the skin in the
aged.", AMERICAN JOURNAL OF CLINICAL
ONCOLOGY APR 2000 LNKD- PUBMED:
10776981, vol. 23, no. 2, April 2000 (2000-04),
pages 181-184, XP009142257, ISSN: 0277-3732**

EP 2 223 683 B1

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

## Description

Field of the Invention

The present invention relates to an oral particulate antitumor preparation coated with a saccharide-based coating film.

Background of the Invention

According to the "Latest Cancer Statistics" published by the Center for Cancer Control and Information Services, National Cancer Center, Japan, the incidence rate of cancer (year 2000) is 11% for men and 10% for women up to age 64, and 27% for men and 17% for women up to age 74. Furthermore, according to the "Vital Statistics" published by the Statistics and Information Department, Minister's Secretariat, Ministry of Health, Labour and Welfare, cancer has ranked the first place among the causes of death in Japan since year 1981, and in year 2004, the number of deaths was 320,315, and the death rate was 31.1% of the total number of deaths.

The methods for treating cancer affecting many people as such include, for example, surgical therapy, chemotherapy, radiotherapy, gene therapy, immunotherapy.

Among these, chemotherapy refers to a drug therapy which is carried out by using antitumor agents, and the antitumor agents are formulated into, for example, injections, tablets, capsules, dry syrups, granules.

Many of the therapeutic methods which make use of injections involve slow administration using intravenous drip, and patients are forced to be under restraint for a long time. On the other hand, oral preparations such as capsules and tablets have a great advantage that since patients can take medicine in places other than the hospital, such as at home or at workplace, administration of the preparations does not bring about significant inconvenience in their daily lives. Therefore, for those patients who are not able to receive treatment by long-term hospitalization after surgery, the oral preparations are often used as a means for adjuvant therapy after the patients are discharged from the hospital.

However, in clinical practice, aged people who have high incidence rates of cancer, often have difficulties in swallowing tablets or capsules as a result of the deterioration of the swallowing function. Furthermore, some of the cancer patients cannot physically take tablets or capsules because they are taking in nutrition through a gastric fistula, a feeding tube or the like. For that reason, the current condition is such that pharmacists pulverize tablets or take out the contents of capsules to administer the products as particulate preparations.

In general, since many of the active ingredients exhibiting antitumor effects have such high pharmacological activity that they are classified as deadly poisons, handling of such active ingredients requires full attention. However, in the antitumor preparations prepared by an operation of pulverizing tablets or taking out the contents of capsules, the drugs contained in the preparations are in an exposed state, and therefore there is always a risk of exposure by the drugs which have high pharmacological activity. This exposure is highly likely to affect pharmacists as well as the patients taking the drugs or those assistants helping the patients to take drugs.

Meanwhile, to date, preparations such as powders, dry syrups, fine granules, and granules have been marketed as particulate preparations. However, as an example of such particulate antitumor preparations that has been surface-engineered to prevent the exposure of drug, only a granular preparation provided with an enteric coating is known hitherto (Patent Document 1), and the purpose of coating in this case was mainly to reduce adverse side effects. Thus, a particulate antitumor preparation which is provided with a coating mainly for the purpose of taking the medicine safely, does not exist. It is contemplated that this might be because there lacks the consideration for the patients, medical assistants and medical service workers who handle antitumor agents having high pharmacological activity.

[Patent Document 1] JP-A-04-36237

EP-A-1 757 283 discloses granules comprising tegafur, gimeracil and oteracil potassium in a molar ratio of 1:0.4:1 for use in the treatment of cancer. It differs from the subject-matter of the present application in that the composition is not coated.

Summary of the Invention

Problems to be Solved by the Invention

An object of the present invention is to provide an oral particulate antitumor preparation, which allows safe intake of antitumor agents, handling of which could be in many cases dangerous due to their high pharmacological activity, and has a stability equivalent to that of capsules or tablets.

As used herein, the term "allows safe intake" means that the drug is not exposed, and there is no chance of exposure by the drug during the production and intake of the preparation.

Means for Solving the Problems

In order to address the above-described problem, the inventors of the present invention attempted to produce a particulate antitumor preparation capable of oral administration, using a variety of rapidly dissolving coating bases.

As a result, the risk of exposure by drug was eliminated by coating the drug, but there arose a problem that an increase in the total amount of related substances including novel related substances is recognized, which has not been recognized in conventional formulations, and thus the stability is significantly decreased.

Therefore, obtaining a particulate preparation having the coated surface and having a stability equivalent to conventional formulations by using coating bases which dissolve rapidly has been considered to be impossible. However, the present inventors continuously conducted investigations from various standpoints, and thus obtained the following findings.

(a) If saccharides are used for the coating base instead of water-soluble polymers, a stability equivalent to that of conventional formulations can be maintained.

(b) A coating film of a particulate preparation thus obtainable is less susceptible to physical abrasion, and thus there is no risk of the exposure of drugs at the time of production and intake of the preparation, while the preparation can be safely taken in. Furthermore, since the coating film dissolves rapidly, bioavailability of the preparation does not decrease.

The present invention was accomplished on the basis of the above findings.

Specifically, the present invention provides an oral antitumor preparation in the form of powder or granules in which a particulate composition containing an orally administrable antitumor agent which is an antimetabolite having an antitumor effect is coated with a saccharide other than a cellulose derivative.

As used herein, the term "particulate preparation" refers to a preparation obtained by producing a pharmaceutical product in the form of powder or granules. The particle size distribution of these pharmaceutical products produced in the form of powder or granules serves as the basis for the classification into powder preparation, fine granular preparation, granular preparation, and the like.

Effects of the Invention

According to the present invention, the following superior effects are provided.

(a) If saccharides are used for the coating base instead of water-soluble polymers, a stability equivalent to that of conventional formulations can be maintained.

(b) A coating film of a particulate preparation thus obtainable is less susceptible to physical abrasion, and thus there is no risk of the exposure of drugs at the time of production and intake of the preparation. Furthermore, since the coating film dissolves rapidly, bioavailability of the preparation does not decrease.

(c) Therefore, according to the present invention, antitumor agents which are in many cases difficult to handle due to their high pharmacological activities, can be safely taken in, and an oral particulate antitumor preparation having a stability equivalent to that of capsules or tablets can be obtained.

Detailed Description of the Invention

The antitumor agent according to the present invention is not particularly limited as long as it contains an active ingredient exhibiting an antitumor effect, and includes, for example, alkylating agents, antimetabolites, antitumor antibiotics, antitumor plant component agents.

Specific examples of the alkylating agents include chloroethylamine-based antitumor agents, ethyleneimine-based antitumor agents, and sulfonic acid ester-based antitumor agents.

Specific examples of the antimetabolites include mercaptopurine-based antitumor agents, methotrexate antitumor agents, fluorouracil-based antitumor agents, and cytosine-based antitumor agents.

Specific examples of the antitumor antibiotics include mitomycin C, actinomycin D, bleomycin-based antitumor agents, anthracycline-based antibiotics, and neocartinostatin antitumor agents.

In addition to those, for example, aceglatone, anastrozole, exemestane, fadrozole hydrochloride hydrate, procarbazine hydrochloride, tamoxifen citrate, toremifene citrate, gefitinib, sobuzoxane, tamibarotene, tretinoin, bicalutamide, flutamide, imatinib mesylate, erlotinib hydrochloride, sorafenib tosylate, sunitinib malate are also included.

Among the antitumor agents, orally administrable antitumor agents are used from the viewpoints of efficacy and safety. As used herein, the "orally administrable antitumor agent" is not particularly limited as long as the agent contains an active ingredient being an antimetabolite which exhibits an antitumor effect by taking by mouth, but it is preferred that the agent contain an active ingredient which is currently marketed in the form of oral preparation.

Among the orally administrable antimetabolites, particularly preferred are combination preparations thereof, from the viewpoint of improving the drug stability. The antimetabolites include, for example, mercaptopurine, methotrexate, capecitabin, carmofur, tegafur, doxifluridine, fluorouracil, cytarabine ocphosphate, hydroxycarbamide, and tegafur/uracil and tegafur/gimeracil/oteracil potassium combination preparations. Among them, a tegafur/gimeracil/oteracil potassium combination preparation is particularly preferred, and a combination preparation in which the molar ratio of the components is 1:0.4:1, is more preferred.

The oral particulate antitumor preparation of the present invention is produced by coating a particulate composition containing the aforementioned antitumor agents, with a saccharide other than a cellulose derivative.

The saccharide according to the present invention is not particularly limited as long as it is a species generally used as an excipient for pharmaceutical products, and is not a cellulose derivative, and includes, for example, monosaccharides, oligosaccharides, polysaccharides.

Examples of the monosaccharides include trioses (such as glyceraldehyde, dihydroxyacetone), tetroses (erythrose, threose, and the like), pentoses (xylose, arabinose, ribose, deoxyribose, and the like), hexoses (glucose, fructose, galactose, mannose, and the like), deoxy sugars (fucose, rhamnose, thioglucose, and the like), amino sugars (glucosamine, galactosamine, and the like), sugar alcohols (mannitol, inositol, and the like), uronic acids (glucuronic acid, galacturonic acid, and the like), and aldonic acids (gluconic acid, and the like).

Examples of the oligosaccharides include disaccharides (trehalose, kojibiose, nigerose, maltose, isomaltose, melibiose, sophorose, laminaribiose, gentiobiose, cellobiose, lactose, turanose, sucrose, leucrose, palatinose, and the like), trisaccharides (6-kestose, 1-kestose, neokestose, melezitose, raffinose, panose, isopanose, lactosucrose, and the like), tetrasaccharides (stachyose, scorodose, and the like), pentasaccharides (verbacose, and the like), and others (cyclodextrin, cyclofructan, cyclodextran, and the like).

Examples of the polysaccharides include starch, agarose, carrageenan, and chitin.

In addition, these saccharides may be used individually alone, or in combination of two or more species.

Among the saccharides, monosaccharides or oligosaccharides are preferred from the viewpoints of an improvement of the drug stability and the physical friability of the coating film, and particularly, sugar alcohols or disaccharides are preferred, while mannitol or sucrose is more preferred.

According to the present invention, cellulose and cellulose derivatives are excluded from the saccharides, and those excluded cellulose derivatives include methylcellulose, ethylcellulose, hydroxypropylcellulose, hypromellose, and the like.

The particulate composition which is to be coated with these saccharides includes a composition obtained by granulating an antitumor agent and known preparation excipients using a conventional granulation method. Examples of the granulation method include the methods for granulating core particles by using a fluidized bed granulation method, a high shear granulation method, a rotary fluidized bed granulation method, an extrusion granulation method, a spray drying granulation method or the like.

Typical methods for producing a particulate composition that can be used in the present invention include, for example, a method of granulating core particles containing an orally administrable antitumor agent, with preparation excipients, by using an extrusion granulation apparatus.

If the aforementioned preparation excipients are to be used, they are used within the scope of not impairing the effects of the present invention. Such preparation excipients are not particularly limited as long as they are various preparation excipients that are generally used in the production of particulate preparations, and includes, for example, fillers, disintegrants, binders, lubricants, coloring agents, flavoring agents, taste improvers. Examples of the fillers include saccharides, light anhydrous silicic acid, and calcium silicate. Examples of the disintegrants include low-substituted hydroxypropylcellulose, carmellose, crospovidone, carmellose calcium, and croscarmellose sodium. Examples of the binders include hydroxypropylcellulose, hypromellose, polyvinyl alcohol, and polyvinylpyrrolidone. Examples of the lubricants include magnesium stearate, calcium stearate, talc, and sucrose ester of fatty acids. Examples of the coloring agents include Food Yellow No. 5, Food Red No. 2, Food Blue No. 2, food lake colors, yellow ferric oxide, and titanium oxide. Examples of the flavoring agents include various flavors such as orange and lemon. Examples of the taste improvers include L-menthol, camphor, and mint.

The coating methods for the obtained granulation product (particulate composition) include, for example, methods using, for example, a fluidized bed, a coating pan.

More preferably, a method of spray coating core particles with an aqueous solution containing a saccharide dissolved therein, by using a fluidized bed, is included.

Furthermore, the proportion of the coating based on a saccharide is not particularly limited as long as the proportion is within the scope of not impairing the effects of the present invention, but the proportion is preferably 70% to 100% by mass, and particularly preferably 90% to 100% by mass, based on the total amount of coating, while it is particularly preferable if the coating is achieved only with saccharides.

The amount of coating is not particularly limited as long as the amount is within the scope of not impairing the effects of the present invention, but the amount is preferably 1% to 20% by mass, more preferably 2% to 15% by mass, and particularly preferably 3% to 10% by mass, based on the total amount of the particulate antitumor preparation.

The dosage form of the particulate antitumor preparation of the present invention is not particularly limited, and includes, for example, granular preparations, powder preparations and fine granular preparations. Furthermore, the granular preparations, powder preparations and fine granular preparations also include dry syrups which can be used by dissolving at the time of use, and also include particulate preparations which dissolve or disintegrate rapidly in the oral cavity and thus can be taken in without water. Here, the particle size of the particulate preparation according to present invention is preferably 75 μm to 1400 μm, and in the case of a granular preparation, more preferably 250 μm to 1000 μm.

According to the present invention, in the case where the preparation contains tegafur as the antitumor agent, the minimum amount of tegafur at a single administration is expected to be about 20 mg; however, if the drug concentration is lower than 0.5% by mass, it is necessary to take more than 4 g of the particulate preparation to take in an amount equivalent to 20 mg of tegafur, and thus intake of the preparation would be more difficult. If the drug concentration is higher than 15% by mass, the amount of preparation equivalent to 20 mg of tegafur will be smaller than 133 mg, but this amount of it would be very difficult to be charged in a generally used dispenser. Thus, it is preferable to include tegafur into the particulate preparation coated with a coating film in an amount of 0.5% to 15% by mass, and particularly preferably 5% to 10% by mass.

The oral particulate antitumor preparation of the present invention is an oral preparation which contains an antitumor agent, and if necessary, the above-described preparation excipients, and which, since has been surface-engineered to prevent the drug from being exposed at the surface, has no risk of drug exposure and can be safely taken in.

Examples

Hereinafter, the present invention will be described in more detail by way of Examples and Test Examples, but the present invention is not limited to these Examples only.

Example 1

150 g of tegafur, 43.5 g of gimeracil, 147 g of oteracil potassium, 2659.5 g of lactose, and 60 g of hydroxypropylcellulose (trade name: "HPC-M", manufactured by Nippon Soda Co., Ltd.) were charged into a kneader (apparatus name: "Dalton Versatile Mixer 25AM-02-rr", manufactured by Dalton Co., Ltd.), and 300 g of purified water was added thereto. The mixture was kneaded for 5 minutes at a rotation speed of 75 min$^{-1}$. This kneaded product was granulated using an extrusion granulator equipped with a screen of ϕ 0.5 mm (apparatus name: "Pelleter Double EXD-60", manufactured by Fuji Paudal Co., Ltd.). This granulated product was sieved with a sieve having a mesh size of 1.5 mm, and then dried using a fluidized bed (apparatus name: "Multiplex MP-01", manufactured by Powrex Corporation). After the drying, the obtained granulated product was sieved to a size range of 355 μm to 1000 μm, to thus obtain a granular preparation.

Comparative Example 1

23.5 g of hypromellose (substitution type 2910, trade name: TC-5R), and 5.9 g of talc were added to 441 g of purified water to prepare a coating solution. 800 g of the granules of Example 1 was charged into a fluidized bed (Multiplex MP-01), and the aforementioned coating solution was sprayed thereon at a spray speed of 3.2 g/min, to obtain coated granules.

Comparative Examples 2 to 4

The water-soluble polymer indicated in Table 1 was used in the amount indicated in Table 1 according to the same method as in Comparative Example 1, to obtain coated granules.

Test Example 1

The granules obtained in Example 1 and Comparative Examples 1 to 4 were stored at 60°C for 10 days, and then the total amount of any related substances generated therefrom was measured for the respective samples according to the liquid chromatography method listed in the Japanese Pharmacopoeia, under the section General Test Methods: Physical Test Methods.

[Table 1]

| | Unit: parts by mass | | | | |
|---|---|---|---|---|---|
| | Example | Comparative Example | | | |
| | 1 | 1 | 2 | 3 | 4 |
| Tegafur | 5.00 | 5.00 | 5.00 | 5.00 | 5.00 |
| Gimeracil | 1.45 | 1.45 | 1.45 | 1.45 | 1.45 |
| Oteracil potassium | 4.90 | 4.90 | 4.90 | 4.90 | 4.90 |
| Lactose | 88.65 | 88.65 | 88.65 | 88.65 | 88.65 |
| Hydroxypropylcellulose | 2.00 | 2.00 | 2.00 | 2.00 | 2.00 |
| Talc | - | 0.75 | 0.75 | 0.75 | 0.75 |
| Hypromellose <TC-5R> | - | 3.00 | - | - | - |
| Hydroxypropylcellulose <HPC-SL> | - | - | 3.00 | - | - |
| Methylcellulose <SM-4> | - | - | - | 3.00 | - |
| Polyvinyl alcohol/ polyethylene glycol/ graft copolymer <Kollicoat IR> | - | - | - | - | 3.00 |
| Total amount of related substances (%) | 0.30 | 1.12 | 0.86 | 1.14 | 1.77 |

As is obvious from the results of Table 1, it was found that when a granular preparation is coated with a water-soluble polymer, the total amount of related substances is increased, and thereby the stability is decreased.

Example 2

300 g of tegafur, 87 g of gimeracil, 294 g of oteracil potassium, 2319 g of lactose, and 60 g of hydroxypropylcellulose (trade name: "HPC-L", manufactured by Nippon Soda Co., Ltd.) were charged into a kneader (apparatus name: "Dalton Versatile Mixer 25AM-02-rr"), and 300 g of purified water was added thereto. The mixture was kneaded for 10 minutes at a rotation speed of 75 min$^{-1}$. This kneaded product was granulated using an extrusion granulator equipped with a screen of $\phi$ 0.5 mm (apparatus name: "Pelleter Double EXD-60"). This granulated product was dried using a fluidized bed (apparatus name: "Multiplex MP-01"). After the drying, the obtained granulated product was sieved to a size range of 250 $\mu$m to 1000 $\mu$m, to thus obtain a granular preparation.
On the other hand, 15 g of sucrose was dissolved in 135 g of purified water to prepare a coating solution. 500 g of the above-described granules was charged into a fluidized bed (Multiplex MP-01), and the coating solution was sprayed thereon at a spray speed of 9 g/min, to obtain coated granules.

Example 3

25 g of D-mannitol was dissolved in 225 g of purified water according to the same method as in Example 2, to prepare a coating solution. 500 g of the aforementioned granules was charged into a fluidized bed (Multiplex MP-01), and the coating solution was sprayed thereon at a spray speed of 12 g/min, to obtain coated granules.

Comparative Example 5

Granules without coating as comparative sample were obtained in the same method as in Example 2, except that the coating with sucrose was not performed.

Test Example 2

The granules obtained in Examples 2 and 3 and Comparative Example 5 were packed in moisture resistant packaging (HDPE bottle + silica gel) and stored at 40°C 75% R.H. for 6 months. Then, the amount of related substances generated therefrom for the respective samples was measured according to the liquid chromatography method listed in the Japanese Pharmacopoeia, under the section General Test Methods: Physical Test Methods.

[Table 2]

|  | Example | | Comparative Example | Capsule preparation |
|---|---|---|---|---|
|  | 2 | 3 | 5 |  |
| Total amount of related substances (%) | 0.29 | 0.23 | 0.24 | 0.30 |

For comparison, the total amount of related substances in a moisture resistant packaged (PTP: Press Through Package + aluminum pouch) commercialized product (tegafur/gimeracil/oteracil capsule, marketed name: "TS-1 Capsule") is presented as well. As is obvious from the results of Table 2, it was found that although a coating of sucrose or D-mannitol is provided to a granular preparation, there is no significant difference in the total amount of related substances. In addition, between sucrose and D-mannitol, the D-mannitol results in a smaller total amount of related substances, even though a larger amount of coating is provided.

Test Example 3

The friability of the coated granules obtained in Examples 2 and 3 was evaluated according to the following evaluation method.

Evaluation method:

About 30 g of coated granules which had been sieved to a particle size of 355 $\mu$m to 710 $\mu$m, was placed, together with four balls made of alumina, in a planetary ball mill (Pulverisette 5, manufactured by Fritsch GmbH) equipped with a pot made of alumina, and the ball mill was operated for 10 minutes at a rotation speed level 6. The granules were removed from the pot, and were sieved with a sieve having a mesh size of 250 $\mu$m. Here, the friability was calculated by the following equation.

$$\texttt{Friability A (\%) = (feed amount - mass on the sieve}$$
$$\texttt{with a mesh size of 250 } \mu\texttt{m)/feed amount} \times \texttt{100}$$

In the case of coated granules, a smaller friability means that the granule surfaces are less defacement, and exposure by drug can be prevented.

[Table 3]

|  | Example | |
|---|---|---|
|  | 2 | 3 |
| Friability (=A) (%) | 2.1 | 2.6 |

The friability of a commercialized product tegafur granule (marketed name: "Sterozine Granules", manufactured by Kotobuki Pharmaceutical Co., Ltd.), as measured by the above-described method of measurement, was 7.4%. As is obvious from the results of Table 3, it was found that the coated granules of Examples 2 and 3 mostly were not defaced, as compared to the existing granular preparation.

Test Example 4

The granules obtained in Examples 2 and 3 were subjected to the dissolution test method listed in the Japanese Pharmacopoeia, under the section General Test Methods: Preparation Test Methods.

<Measurement conditions>

Dissolution test method: Second method (50 min$^{-1}$)
Test medium: water (900 mL)
Measurement wavelength: $\lambda$ = 262 nm
Sampling: 5 minutes after initiation

[Table 4]

| Name of ingredient | Example | |
| --- | --- | --- |
| | 2 | 3 |
| Dissolution rate of tegafur (%) | 93.8 | 94.5 |
| Dissolution rate of gimeracil (%) | 84.2 | 83.7 |
| Dissolution rate of oteracil potassium (%) | 93.4 | 94.0 |

As is obvious from the results of Table 4, most of the active ingredients (tegafur, gimeracil and oteracil potassium) were dissolved in 5 minutes from the coated granules of Examples 2 and 3.

According to the above results, it was found that when a saccharide is used for the coating base instead of a water-soluble polymer, a stability equivalent to that of conventional formulations can be maintained, and the coating film is rapidly dissolved and the drug is rapidly released, so that there is no risk of decreased bioavailability. Furthermore, since the physical abrasion of the coating film occurs less, there is no risk of drug exposure during the production and intake of the preparation, and the preparation can be safely taken in.

**Claims**

1.  Oral antitumor preparation in the form of powder or granules, comprising a particulate composition containing an orally administrable antitumor agent which is an antimetabolite having an antitumor effect, wherein the particulate composition is coated with a saccharide other than a cellulose derivative.

2.  The oral antitumor preparation in the form of powder or granules according to claim 1, wherein the saccharide is a monosaccharide or an oligosaccharide.

3.  The oral antitumor preparation in the form of powder or granules according to claim 1 or 2, wherein the saccharide is a sugar alcohol or a disaccharide.

4.  The oral antitumor preparation in the form of powder or granules according to any one of claims 1 to 3, wherein the saccharide is mannitol or sucrose.

5.  The oral antitumor preparation in the form of powder or granules according to any one of claims 1 to 4, having a dosage form of a granular preparation.

6.  The oral antitumor preparation in the form of powder or granules according to claim 1, wherein the antitumor agent contains tegafur.

7.  The oral antitumor preparation in the form of powder or granules according to claim 1 or 6, wherein the antitumor agent contains (a) tegafur, (b) gimeracil, and (c) oteracil potassium.

8.  The oral antitumor preparation in the form of powder or granules according to claim 7, comprising (a) tegafur, (b) gimeracil, and (c) oteracil potassium in a molar ratio of 1:0.4:1.

9.  The oral antitumor preparationin the form of powder or granules according to any one of claims 1 to 8, comprising 0.5% to 15% by mass of tegafur in the particulate preparation coated with a coating film.

10. The oral antitumor preparation in the form of powder or granules according to any one of claims 1 to 9, comprising 5% to 10% by mass of tegafur in the particulate preparation coated with a coating film.

**Patentansprüche**

1. Oral einnehmbares Antitumormittel in Form von Pulver oder Granulat, umfassend eine teilchenförmige Zusammensetzung, enthaltend ein oral einnehmbares Antitumormittel, das ein Anitmetabolit mit einem Antitumoreffekt ist, wobei die teilchenförmige Zusammensetzung mit einem Saccharid, das kein Zellulosederivat ist, beschichtet ist.

2. Das oral einnehmbare Antitumormittel in Form von Pulver oder Granulat nach Anspruch 1, wobei das Saccharid ein Monosaccharid oder ein Oligosaccharid ist.

3. Das oral einnehmbare Antitumormittel in Form von Pulver oder Granulat nach Anspruch 1 oder 2, wobei das Saccharid ein Zuckeralkohol oder ein Disaccharid ist.

4. Das oral einnehmbare Antitumormittel in Form von Pulver oder Granulat nach einem der Ansprüche 1 bis 3, wobei das Saccharid Mannitol oder Saccharose ist.

5. Das oral einnehmbare Antitumormittel in Form von Pulver oder Granulat nach einem der Ansprüche 1 bis 4 in einer Dosierungsform einer Granulatzubereitung.

6. Das orale Antitumormittel in Form von Pulver oder Granulat nach Anspruch 1, wobei das Antitumormittel Tegafur enthält.

7. Das oral einnehmbare Antitumormittel in Form von Pulver oder Granulat nach einem der Ansprüche 1 bis 6, wobei das Antitumormittle (a) Tegafur, (b) Gimeracil, und (c) Oteracil Kalium enthält.

8. Das oral einnehmbare Antitumormittel in Form von Pulver oder Granulat nach Anspruch 7, umfassend (a) Tegafur, (b) Gimeracil und (c) Oteracil Kalium in einem Molverhältnis von 1:0,4:1.

9. Das oral einnehmbare Antitumormittel in Form von Pulver oder Granulat nach einem der Ansprüche 1 bis 8, umfassend 0,5 bis 15 Massen-% Tegafur in der teilchenförmigen Zubereitung, die mit einem Beschichtungsfilm beschichtet ist.

10. Das oral einnehmbare Antitumormittel in Form von Pulver oder Granulat nach einem der Ansprüche 1 bis 9, umfassend 5 bis 10 Masse-% Tegafur in der teilchenförmigen Zubereitung, die mit einem Beschichtungsfilm beschichtet ist.

**Revendications**

1. Préparation antitumorale orale sous la forme de poudre ou de granules, comprenant une composition particulaire contenant un agent antitumoral administrable par voie orale qui est un antimétabolite présentant un effet antitumoral, étant précisé que la composition particulaire est recouverte d'un saccharide autre qu'un dérivé de la cellulose.

2. Préparation antitumorale orale sous la forme de poudre ou de granules selon la revendication 1, étant précisé que le saccharide est un monosaccharide ou un oligosaccharide.

3. Préparation antitumorale orale sous la forme de poudre ou de granules selon la revendication 1 ou 2, étant précisé que le saccharide est un sucre-alcool ou un disaccharide.

4. Préparation antitumorale orale sous la forme de poudre ou de granules selon l'une quelconque des revendications 1 à 3, étant précisé que le saccharide est du mannitol ou du saccharose.

5. Préparation antitumorale orale sous la forme de poudre ou de granules selon l'une quelconque des revendications 1 à 4, présentant la forme pharmaceutique d'une préparation granulaire.

6. Préparation antitumorale orale sous la forme de poudre ou de granules selon la revendication 1, étant précisé que l'agent antitumoral contient du tégafur.

7. Préparation antitumorale orale sous la forme de poudre ou de granules selon la revendication 1 ou 6, étant précisé que l'agent antitumoral contient (a) du tégafur, (b) du giméracil et (c) de l'otéracil potassium.

8. Préparation antitumorale orale sous la forme de poudre ou de granules selon la revendication 7, comprenant (a) du tégafur, (b) du giméracil et (c) de l'otéracil potassium selon un rapport molaire de 1:0.4:1.

9. Préparation antitumorale orale sous la forme de poudre ou de granules selon l'une quelconque des revendications 1 à 8, comprenant 0,5 % à 15 % en masse de tégafur dans la préparation particulaire recouverte d'une pellicule protectrice.

10. Préparation antitumorale orale sous la forme de poudre ou de granules selon l'une quelconque des revendications 1 à 9, comprenant 5 % à 10 % en masse de tégafur dans la préparation particulaire recouverte d'une pellicule protectrice.

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 4036237 A
- EP 1757283 A